# EUROPEAN PATENT APPLICATION

(11) **EP 2 484 692 A1**
(43) Date of publication of application: **08.08.2012**
(21) Application number: 09850086.1
(22) Date of filing: 30.09.2009
(51) Int. Cl.: C07K 14/775, G01N 33/68, A61K 38/17, A61P 3/06

(54) **APOLIPOPROTEIN A-1 MIMIC PEPTIDES, AND THERAPEUTIC AGENT FOR TREATING HYPERLIPIDEMIA AND DISEASES RELATED TO HYPERLIPIDEMIA COMPRISING SAME**

(71) Applicant: SNU R&DB Foundation, Seoul 151-742 (KR)
(72) Inventor: YU, Jaehoon, Seoul 135-280 (KR)
(74) Representative: Office Freylinger
(86) International application number: PCT/KR2009/005607
(87) International publication number: WO 2011/040654

(57) **Abstract**

The present invention relates to apolipoprotein A-1 mimic peptides, and therapeutic agent for treating hyperlipidemia and diseases related to hyperlipidemia comprising the same. More specifically, the apolipoprotein A-1 mimic peptides of the present invention were manufactured by modifying hydrophilic or hydrophobic face of existing 4F amphipathic peptides to produce Apo A-I mimic peptides which specifically bind with cholesterol ester to allow high density lipoprotein content to increase, and the peptide of which phenylalanine in hydrophobic face of 4F is substituted with 2-naphtilalanine has superior cholesterol efflux capability and cognitive function for lipids to the existing 4F peptides, among the mimic peptides. Thus, the Apo A-I mimic peptides of the present invention can be used as Apo A-I mimic peptides and as a therapeutic agent for treating hyperlipidemia and diseases related to hyperlipidemia.

## Description

### TECHNICAL FIELD

The present disclosure relates to apolipoprotein A-1 (Apo-1) mimic peptides, and therapeutic agent for treating hyperlipidemia comprising the same.

### BACKGROUND ART

Hyperlipidemia is a condition in which an excessive amount of fatty materials circulate in the blood and can accumulate in the walls of the arteries, causing an inflammatory response and leading to cardiovascular disease. The definition of hyperlipidemia is when the concentration of any of the serum lipids such as cholesterol, triglyceride, phospholipid and free fatty acid is higher than the healthy fasting range. The healthy fasting triglyceride level is 50-150 mg/dl, phospholipid is 150-250 mg/dl, cholesterol is 130-230 mg/dl and free fatty acid is 5-10 mg/ml. When left unattended, hyperlipidemia can increase the risk of serious complications such as high blood pressure, atherosclerosis (angina, myocardial infarction) and cerebral arteriosclerosis (cerebral infarction).

The increase in low density lipoprotein (LDL) is an independent risk factor for hyperlipidemia. LDL is one of the molecules that transports cholesterol in the liver or intestine to tissues. LDL is also known as "bad cholesterol". Since LDL contains high levels of cholesterol, it can accumulate on artery walls when the level of LDL increases in the blood stream, leading to an increased risk of coronary artery disease and heart attack. Therefore, several therapeutic agents that can reduce the level of LDL have been developed to treat hyperlipidemia and related diseases. However, recent studies have shown that increasing the level of high density lipoprotein (HDL) rather than lowering LDL has greater effect on treating hyperlipidemia. Therefore, drugs which aim to increase HDL levels are being developed.

High density lipoprotein (HDL) is generated through aggregation of cholesterol or cholesterol esters released from macrophages near capillaries, with the Apolipoprotein A-I (Apo-I) in the blood stream. Unlike LDL, HDL picks up cholesterol from the tissue and carries it in the blood stream to liver to be absorbed and degraded. Therefore, when this process is repeated, the cholesterol level in macrophages located near the capillaries will decrease, leading to a reduction in arterial macrophage sizes and expansion of the blood vessels. When reverse cholesterol transport is activated by increasing the level of HDL in the blood stream, this will have the effect of reducing the blood cholesterol level in the blood stream or in macrophages. Therefore, the increase in serum HDL concentration may be an important factor in treating hyperlipidemia or coronary artery disease in patients.

Until recently, only two targets which can increase HDL have been discovered. One is Apo A-I, which helps the cholesterol or cholesterol esters released from macrophage cells to aggregate. The Apo A-I protein is formed by several alpha-helices, which surround the outside of the unstable cholesterol deposits to stabilize them as HDL. Therefore, if a compound that mimics Apo A-I exists in the blood, this could increase the property of HDL in the blood. Two types of peptide which mimics Apo A-I have been proven to have a therapeutic effect in phase 1 and further clinical trials.

Another target protein is cholesterol ester transfer protein (CETP). This protein induces the conversion of HDL to LDL. Therefore, CETP inhibitors, which can inhibit the function of this protein has been developed by major pharmaceutical companies like Pfizer and Merck. However, Torcetrapib, the leading CETP inhibitor of Pfizer Pharmaceuticals failed clinical trials in year 2006. This raised concerns about possible side effects of CETP inhibitors besides blocking conversion of HDL to LDL, and lost its potential as the therapeutic drug target to increase HDL.

After the failure of CETP inhibitors, Apo A-I protein and its mimic peptides gained interest as target and drug for increasing HDL levels. Two drug candidates mimicking ApoA-1 protein that completed the phase 1 clinical trials are Apo A-Iₘᵢₗₐₙₒ protein, which mimics part of the Apo A-I protein and amphiphilic peptide D-4F, which has a different amino acid sequence than Apo A-I but readily forms alpha helices. These compounds entered phase 3 and phase 2 clinical trials, respectively, in the end of 2007.

D-4F consists of 18 D-amino acids that forms a α-helical shape, and was developed for oral administration. In the hydrophilic face, half of the amino acid is positively charged lysine and the rest is negatively charged glutamic acid or aspartic acid. When this peptide forms a α-helix, other peptide molecules can form layers by interacting with the positive and negative charges of their peptide to form macromolecule. The molecular assembly occurs by alpha helical peptides functioning as a single subunit of the fence, lining up in the form of a fence. Molecular recognition is processed as if making a fence around the cholesterol. Therefore, the positively and negatively charged amino acid residues in the hydrophilic face of the peptide are considered to have an important function. There were several experiments on improving the hydrophilic part of D-4F by changing the position of positively and negatively charged amino acid residues. However, no peptide has been reported to have a better effect than D-4F. In addition, there has been no attempt to modifying D-4F by using artificial amino acid to improve D-4F.

However, the 4 phenylalanine residues positioned at the hydrophilic face of the D-4F peptide directly interact with the cholesterol, therefore the position and the shape of phenylalanine is considered to be important. 4F (composed of 18 L-amino acids) peptide synthesized earlier than D-4F indicated the importance of the numbers and the position of phenylalanine. The peptide function decreased when the number of phenylalanine was either more or less than 4. It is considered that phenylalanine is usually involved in recognition of cholesterol or cholesterol esters, which are hydrophobic molecules. However, there have been no attempts to improve 4F material by using more complicated aromatic amino acids or artificial aromatic amino acids, in place of simple phenylalanine groups.

The present inventors have conducted intensive research to develop a peptide which can treat hyperlipidemia and related diseases by increasing HDL more effectively than existing peptide. As a result, the present inventors constructed a peptide library by modifying the hydrophilic and hydrophobic face of 4F [amphiphilic alpha helix peptide containing 4 phenylalanine (F) that binds to Apo A-I like lipids] using non-natural amino acids rather than well known naturally occurring amino acids. By screening the library, the present inventors confirmed that Apo A-I mimic peptide has superior function in cholesterol efflux capability and recognition of hydrophobic molecules, and therefore can be an effective therapeutic agent for treating hyperlipidemia and related diseases by effectively transporting the cholesterol to the liver for excretion, thus completed the present invention.

### DISCLOSURE OF THE INVENTION

### TECHNICAL PROBLEM

One object of the present invention is to provide apolipoprotein A-1 (Apo-1) mimic peptides, and therapeutic agent for treating hyperlipidemia comprising the same.

### TECHNICAL SOLUTION

In order to achieve the object, the present invention provides an amphipathic peptide library comprising amphipathic peptide sequence containing 3 to 8 phenylanines (F) at one side of the amphipathic α-helical peptide, wherein a positively charged amino acid of the hydrophilic amino acid is substituted with amino acid with less carbon numbers, and a negatively charged amino acid is substituted with amino acid with more carbon numbers.

Also, the present invention provides an amphipathic peptide library comprising amphipathic peptide sequence containing 3 to 8 phenylanines (F) at one side of the amphipathic α-helical peptide, wherein one or more phenylalanine of the hydrophobic amino acid is substituted with aromatic amino acid other than phenylalanine.

The present invention also provides a method of screening Apo A-I mimic peptide, which comprises the steps of:

1) preparing the amphipathic peptide library;

2) analyzing the cholesterol efflux from a cell by the amphipathic peptide library of step 1) ; and

3) selecting peptide which shows increased level of cholesterol efflux than Apolipoprotein A-1(Apo A-I).

Also, the present invention provides a method of screening Apo A-I mimic peptide, which comprises the steps of:

1) preparing the amphipathic peptide library; and

2) selecting peptide which shows increased level of tryptophan fluorescence intensity than Apolipoprotein A-1(Apo A-I) by mixing tryptophan fluorescence probe molecule to the amphipathic peptide library of step 1) and detecting with a fluorescence spectrometer.

The present invention also provides a high density lipoprotein (HDL) enhancing agent comprising the amphipathic peptide selected from the method as an active component.

Also, the present invention provides a therapeutic agent or a diagnostic reagent for hyperlipidemia and related diseases comprising the amphipathic peptide selected from the method as an active component.

Also, the present invention provides a use of amphipathic peptide for manufacturing a therapeutic agent or a diagnostic reagent for hyperlipidemia and related diseases.

Hereinafter, the present invention is described in detail.

The present invention provides an amphipathic peptide library comprising amphipathic peptide sequence containing 3 to 8 phenylanines (F) at one side of the amphipathic α-helical peptide, wherein a positively charged amino acid of the hydrophilic amino acid is substituted with amino acid with less carbon numbers, and a negatively charged amino acid is substituted with amino acid with more carbon numbers.

The amphipathic peptide may include peptide containing D-amino acid, but not limited thereto.

As one example of the amphipathic peptide library of the present invention, the present invention may comprise amphipathic peptide containing amino acid sequence (4F) represented by SEQ ID NO: 1, wherein one or two of the hydrophilic amino acid lysine (K) is substituted with ornithine (Orn), 1,4-diaminobutyric acid (Dab) and 1,3-dipropanoic acid (Dap), and one or two of the amino acid glutamic acid (E) or aspartic acid (D) is substituted with glutamic acid or amino adipic acid (Aad), but not limited thereto.

Preferably, the amphiphilic peptide library may include one or more peptides having the amino acid sequences represented by SEQ. ID. NOs: 3 to 24, and more preferably include SEQ. ID. NO: 3, but not limited thereto.

Also, the present invention provides an amphipathic peptide library comprising amphipathic peptide sequence containing 3 to 8 phenylanines (F) at one side of the amphipathic α-helical peptide, wherein one or more phenylalanine of the hydrophobic amino acid is substituted with aromatic amino acid other than phenylalanine.

The amphipathic peptide may include peptide containing D-amino acid, but not limited thereto.

As one example of the amphipathic peptide library of the present invention, the present invention provides the amphipathic peptide containing amino acid sequence (4F) represented by SEQ ID NO:1, wherein one or two of the hydrophobic amino acid phenylalanine(F) is substituted with alanine (A), tryptophan (W), 1-naphthylalanine (Nal1) or 2-naphthylalanine (Nal2).

Preferably, the amphiphilic peptide library may include one or more peptides having the amino acid sequence represented by SEQ. ID. NOs: 25 to 44, and more preferably include SEQ. ID. NO: 43, but not limited thereto.

The present inventors prepared a peptide that maintains the α-helical structure by maintaining the important amino acid sequence of 4F, while substituting the hydrophilic or hydrophobic amino acid residues, in order to discover an Apo A-I mimic peptide with improved recognition for cholesterol while maintaining the α-helices of the existing Apo A-I mimic peptide 4F.

Most of the amino acids in the hydrophilic region are positively charged lysine (K), and negatively charged aspartic acid or glutamic acid. The recognition between peptide molecules will occur through this positive charge-negative charge recognition. Based on the hypothesis that the recognition can be fine tuned by controlling the carbon length of the charged amino acid, the recognition difference was studied by shortening the positively charged amine functional groups while increasing the number of carbons in the negatively charged acidic region. Also, two of the positively charged or negatively charged amine functional group or acid functional group located in close vicinity is likely to recognize each other, therefore peptide was prepared by changing the carbon number of the two amino acid residues which were in the shortest distance to each other. Hydrophilic amino acid lysine (K, contains 4 carbons from α-carbon) were substituted with ornithine (Orn, contains 3 carbons from α-carbon), diaminobutyric acid (Dab, contains 2 carbons from α-carbon) or diaminopropionic acid (Dap, contains 1 carbons from α-carbon), or aspartic acid (D) was substituted with glutamic acid (E, contains 1 more carbon than aspartic acid) or aminoadipic acid (Aad, contains 2 more carbon than aspartic acid)(SEQ. ID. NOs:3 24).

Regarding the hydrophobic region, phenylalanine (F) was mainly substituted, which is known to be an important factor in existing Apo A-I mimic peptide 4F. In detail, peptide in which 4 phenylalanines (F) at the hydrophobic face is substituted with alanine (A) (SEQ. ID. NOs: 25 to 28), peptide in which phenylalanine is substituted with tryptophan (W)SEQ. ID. NOs: 25 to 28), peptide in which phenylalanine is substituted with 1-naphthylalanine(Nal1)(SEQ. ID. NOs: 33 to 36), peptide in which phenylalanine is substituted with 2-naphthylalanine(Nal2)(SEQ. ID. NOs: 37 to 40), and peptide in which phenylalanine is substituted with 1-naphthylalanine and 2-naphthylalanine (SEQ. ID. NOs: 41 to 44) were prepared, and an amphipathic peptide library comprising one or more of the amphipathic peptide was constructed.

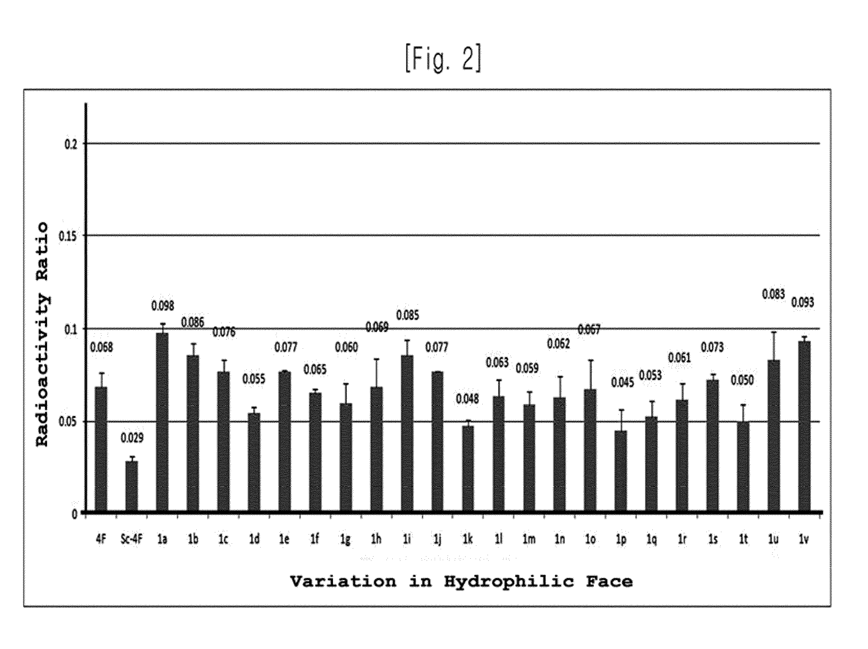

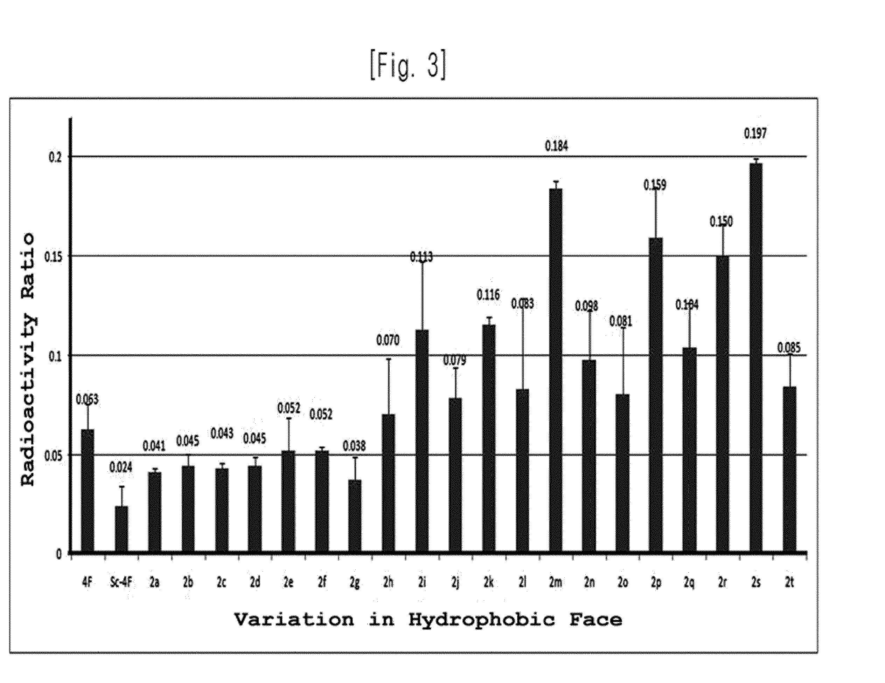

The present inventors determined the calculated mass value of the Apo A-I mimic peptide and the actual mass value of the A-1 mimic peptide acquired following the synthesis and purification step (see Table 1). In addition, cholesterol efflux was analyzed to screen the peptides. Human macrophage cells were treated with ³H radioisotope labeled cholesterol. Next, the cells were treated with each of the peptide prepared from above. The amount of high density lipoprotein (HDL) produced was analyzed by measuring the amount of intracellular cholesterol and extracellular cholesterol released from the macrophage. As a result, hydrophilicity modified peptide showed similar or relatively higher effect when compared to 4F (see FIG.2). 1a (SEQ. ID. NO: 3) showed about 150% higher level of cholesterol efflux when compared to 4F. Hydrophobiciy modified peptide showed significantly higher increase when compared to hydrophilicity modified peptide. In particular, peptide 2s, in which the phenylalanine residues at position 3 and position 18 were substituted with 2-napthylalanine showed over 300% higher increase in cholesterol efflux activity when compared to existing 4F. In addition, 4 peptides in which phenylalanine were substituted with alanine showed reduction in cholesterol efflux. There was a significant increase in cholesterol efflux when phenylalanine was substituted with tryptophan or napthylalanine (see FIG. 3). This result suggests that 4 phenylalanine residues have important function in cholesterol efflux, and phenylalanine at position 3 substituted with tryptophan or napthylalanine shows the most enhanced effect, therefore indicating the hydrophobic residue at this position is important in mimicking Apo A-I protein.

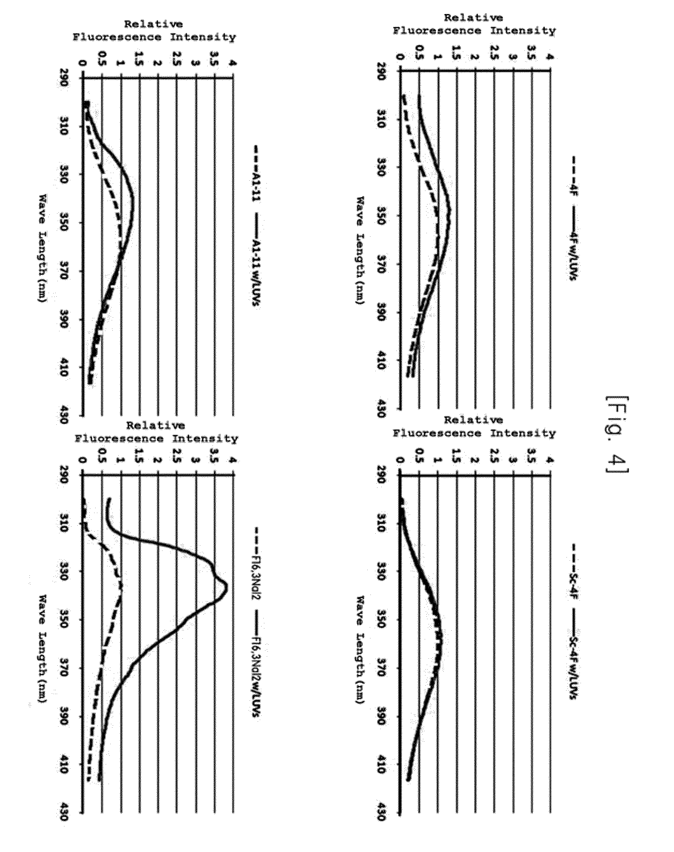

The present inventors analyzed the recognition strength of 1a (150% increase when compared to 4F) or 2s (300% increase when compared to 4F) which were isolated by screening the hydrophilic or hydrophobic face modified peptides that showed high cholesterol efflux effect with the hydrophobic molecules under lipid or aqueous solution. The recognition strength was analyzed by measuring the changes in tryptophan fluorescence intensity of the tryptophan in the peptide. As a result, 2s peptide showed about 4.30-fold increase in fluorescence intensity in the lipid environment when compared to in aqueous solution, and 1a showed about 2.1-fold increased. The result indicated that 2s peptide had a higher effect than 4F peptide (see FIG. 4). Therefore, 2s peptide is confirmed to have strong recognition with surrounding lipids such as cholesterol.

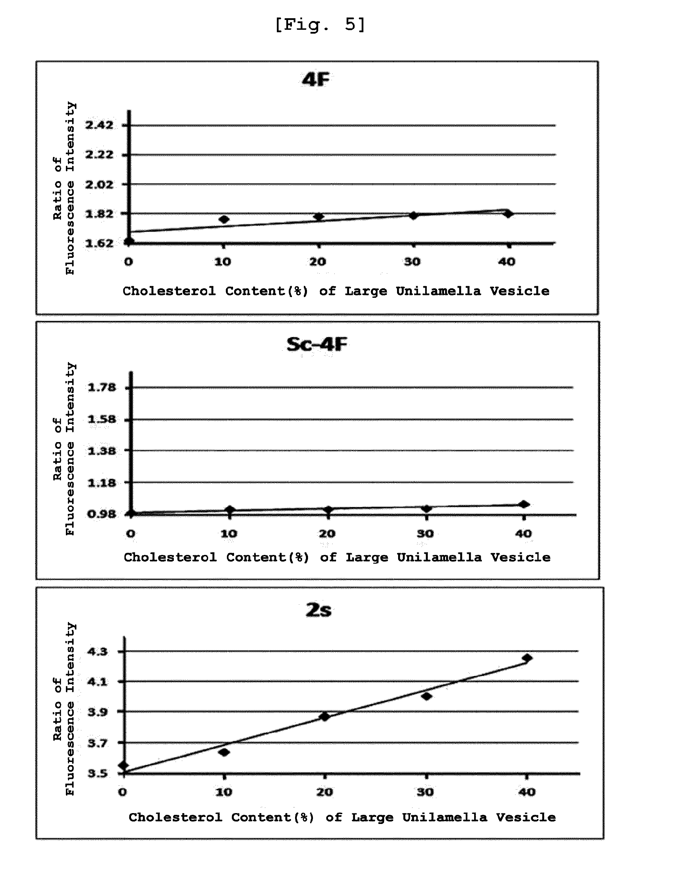

To understand the recognition difference between peptide and cholesterol depending on the concentration of the cholesterol, the increase in fluorescence intensity of the tryptophan containing peptide was analyzed by using liposome with increased relative amounts of cholesterol. As a result, there was an increase in fluorescence intensity depending on the increase of the cholesterol amount, when compared to membrane structure without cholesterol. This result confirmed that there is a close interaction between the cholesterol and the peptide (see FIG. 5).

Therefore, by producing Apo A-I mimic peptide which has superior effect than the existing 4F, the Apo A-I mimic peptide can increase the HDL in the blood stream by selectively recognizing the cholesterol more effectively than the existing 4F, therefore can be an effective therapeutic agent for hyperlipidemia, instead of Apo A-I protein.

Also, the present invention provides a method of screening Apo A-I mimic peptide using the amphipathic peptide library.

In particular, the present invention provides a method of screening Apo A-I mimic peptide, which comprises the steps of:

1) preparing the amphipathic peptide library;

2) analyzing the cholesterol efflux in a cell by the amphipathic peptide library of step 1) ; and

3) selecting peptide showing increased level of cholesterol efflux when compared to Apo A-I.

According to the above method, the cell in step 1) is preferably a macrophage cell, but not limited thereto.

According to the above method, the level of cholesterol efflux of step 2) is analyzed preferably by cholesterol efflux assay, but is not limited thereto.

The amphipathic peptide selected by screening the amphipathic peptide which shows cholesterol efflux level higher than Apo A-peptide may be effectively used as an Apo A-I mimic protein.

Also, the present invention provides another method for screening Apo A-I mimic peptide.

In particular, a method of screening Apo A-I mimic peptide which comprises the steps of:

1) preparing the amphipathic peptide library; and

2) selecting peptide showing increased level of tryptophan fluorescence intensity when compared to Apo A-I by mixing a tryptophan fluorescence probe molecule to the amphipathic peptide library of step 1) and detecting with a fluorescence spectrometer.

According to the above method, recognition strength for lipid or hydrophobic molecule can be calculated from the tryptophan fluorescence intensity of step 2).

The amphipathic peptide selected by screening the amphipathic peptide which shows tryptophan fluorescence intensity higher than Apo A-peptide may be effectively used as Apo A-I mimic protein.

Also, the present invention provides high density lipoprotein (HDL) enhancing agent comprising the amphipathic peptide selected by the method as an active component.

The present invention prepared and screened Apo A-I mimic peptide that can increase high density lipoprotein by modifying the hydrophilic or hydrophobic amino acid of the existing amphipathic peptide, and confirmed that phenylalanine at the hydrophobic face substituted with 2-naphthylalanine has superior function in cholesterol efflux capability and recognition of hydrophobic molecules when compared to 4F, therefore may be effectively used as a HDL enhancing agent.

Also, the present invention provides a therapeutic agent or diagnostic reagent for treating hyperlipidemia and related diseases comprising the amphipathic peptide selected from the method as an active component.

Also, the present invention provides diagnostic reagent for hyperlipidemia and related diseases comprising the amphipathic peptide selected from the method as an active component.

The hyperlipidemia and related diseases are preferably any one selected from the group consisting of hypercholesterolemia, atherosclerosis, coronary artery disease, cardiovascular disease, restenosis, high blood pressure, angina, diabetes, obesity and Alzheimer's disease, but not limited thereto.

The therapeutic agent or the diagnostic reagent may contain Apo A-I mimic peptide, and may further contain peptide modified with D-amino acid.

The present invention prepared and screened Apo A-I mimic peptide that can increase high density lipoprotein by modifying the hydrophilic or hydrophobic amino acid of the existing amphipathic peptide, and confirmed that phenylalanine at the hydrophobic face substituted with 2-naphthylalanine has superior function in cholesterol efflux capability and recognition of hydrophobic molecules compared to 4F, therefore may be effectively used as a therapeutic agent or diagnostic reagent for treating hyperlipidemia and diseases.

Meanwhile, the therapeutic agent and diagnostic reagent comprising the Apo A-I mimic peptide as an active component, may contained Apo A-I mimic peptide in the amount of 0.0001 to 50 wt% based on the total weight of the component.

The therapeutic agent of the present invention may further contain one or more active component which has identical or similar function to the active component.

In the pharmaceutical compositions of this invention, the pharmaceutically acceptable carrier may be existing one for formulation, including saline solution, sterilized water, Ringers solution, buffered saline solution, dextrose solution, maltodextrin solution, glycerin, ethanol, liposome and a mixture of at least one thereof may be used. The pharmaceutical composition according to the present invention may further include a diluent, a dispersing agent, a surfactant, a binder and a lubricant to be prepared into a formation for injection, such as aqueous solution, suspension, emulsion, pill, capsule, granule or tablet. Antibodies and other ligands specific to a target cell may be used in combination with the carrier to be specifically reacted with the target cell. Furthermore, the composition may be preferably formulated according to each disease or ingredient using a suitable method in the art or a method which is taught in Remington's Pharmaceutical Science, Mack Publishing Company, Easton Pa).

The pharmaceutical composition of this invention may be administered orally or parenterally. The method for administration may be conducted by oral or parenteral administration (for example, intravenous, subcutaneous, intraperitoneal, or local or nasal administration) according to the purpose of use, preferably by parenteral administration, and more preferably by nasal administration. A suitable dose of the pharmaceutical composition of the present invention may vary depending on pharmaceutical formulation methods, administration methods, the patient's age, body weight, sex, severity of diseases, diet, administration time, administration route, an excretion rate and sensitivity for a used pharmaceutical composition. The pharmaceutical composition of the present invention is administered with a daily dose of 0.005-10 mg/kg (body weight), preferably 0.05 to 1 mg/kg, and more preferably administered once to several times a day.

The therapeutic agent of the present invention may be used alone or in combination with other methods such as surgery, hormone therapy, chemical therapy and biological response controller for the treatment of the patient.

The present invention also provides a therapy kit for hyperlipidemia or related diseases, comprising the amphipathic peptide selected by the method as an active component.

Also, the present invention provides a diagnosis kit for hyperlipidemia or related diseases, comprising the amphipathic peptide selected by the method as an active component.

The hyperlipidemia and related diseases is preferably any one selected from the group consisting of hypercholesterolemia, atherosclerosis, coronary artery disease, cardiovascular disease, restenosis, high blood pressure, angina, diabetes, obesity and Alzheimer's disease, but not limited thereto.

The present invention prepared and screened Apo A-I mimic peptide that can increase high density lipoprotein by modifying the hydrophilic or hydrophobic amino acid of the existing amphipathic peptide, and confirmed that phenylalanine at the hydrophobic face substituted with 2-naphthylalanine has superior function in cholesterol efflux capability and recognition of hydrophobic molecules when compared to 4F, therefore may be effectively used as a component for therapy and diagnosis kit for treatment and diagnosis of hyperlipidemia and related diseases.

Also, the present invention provides a method of treating hyperlipidemia and related diseases comprising a step of administering the therapeutic agent to a subject.

Also, the present invention provides a method of diagnosing hyperlipidemia and related diseases comprising a step of administering the therapeutic agent to a subject.

The subject applicable in the present invention is a vertebrate, preferably a mammal, more preferably an experimental animal such as mouse, rabbit, guinea pig, hamster, dog and cat, and most preferably a primate such as chimpanzee and gorilla.

The hyperlipidemia and related diseases is preferably any one selected from the group consisting of hypercholesterolemia, atherosclerosis, coronary artery disease, cardiovascular disease, restenosis, high blood pressure, angina, diabetes, obesity and Alzheimer's disease, but not limited thereto.

The present invention prepared and screened Apo A-I mimic peptide that can increase high density lipoprotein by modifying the hydrophilic or hydrophobic amino acid of the existing amphipathic peptide, and confirmed that phenylalanine at the hydrophobic face substituted with 2-naphthylalanine has superior function in cholesterol efflux capability and recognition of lipid when compared to 4F, therefore may be effectively used for treatment and diagnosis of hyperlipidemia and related diseases.

The pharmaceutical composition of the present invention may be parenterally administered, and the parenteral administration is effected by intravenous injection, intraperitoneal injection, subcutaneous injection, intrarectal injection, intracerebroventricular injection or intrathoracic injection. The dose may vary depending on weight, age, sex, and health condition of a patient, diet, administration time, administration method, excretion rate, and severity of disease. The dose may vary depending on weight, age, sex, and health condition of a patient, diet, administration time, administration method, excretion rate, and severity of disease. The single dose is in the range of 0.005 to 10 mg/kg, preferably in the range of 0.05 to 1 mg/kg, which may be administered once to several times a day.

The present invention also provides a use of an amphipathic peptide for manufacturing therapeutic agent or diagnostic reagent for hyperlipidemia and related diseases, wherein the amino acid sequence of amphipathic α-helical peptide with 4 phenylalanines (F) contains a positively charged amino acid of the hydrophilic amino acid substituted with an amino acid with fewer carbon numbers, and a negatively charged amino acid of the hydrophilic amino acid substituted with an amino acid with more carbon numbers.

Also, the present invention provides a use of an amphipathic peptide for manufacturing therapeutic agent or diagnostic reagent for treating hyperlipidemia and related diseases, wherein the amino acid sequence of the amphipathic α-helical peptide with 4 phenylalanines (F) contains one or more phenylalanine of the hydrophobic amino acid substituted with aromatic amino acid other than phenylalanine.

The hyperlipidemia and related diseases is preferably any one selected from the group consisting of hypercholesterolemia, atherosclerosis, coronary artery disease, cardiovascular disease, restenosis, high blood pressure, angina, diabetes, obesity and Alzheimer's disease, but not limited thereto.

The present invention prepared and screened Apo A-I mimic peptide that can increase high density lipoprotein by modifying the hydrophilic or hydrophobic amino acid of the existing amphipathic peptide, and confirmed that phenylalanine at the hydrophobic face substituted with 2-naphthylalanine has superior function in cholesterol efflux capability and recognition of hydrophobic molecules when compared to 4F, therefore the Apo A-I mimic protein may be effectively used as an active component of therapeutic agent for treating hyperlipidemia and related diseases.

### ADVANTAGEOUS EFFECTS

The Apo A-I mimic peptide can be used for screening amphipathic peptide that can increase high density lipoprotein in the blood, and since the selected 4F peptide in which the phenylalanine of the hydrophobic face was substituted with 2-naphthylalanine showed superior function in cholesterol efflux capability and recognition of lipid when compared to 4F, therefore may be effectively used as a Apo A-I mimic peptide for treating hyperlipidemia and related diseases and as a candidate peptide for therapeutic agents.

### BRIEF DESCRIPTION OF THE DRAWINGS

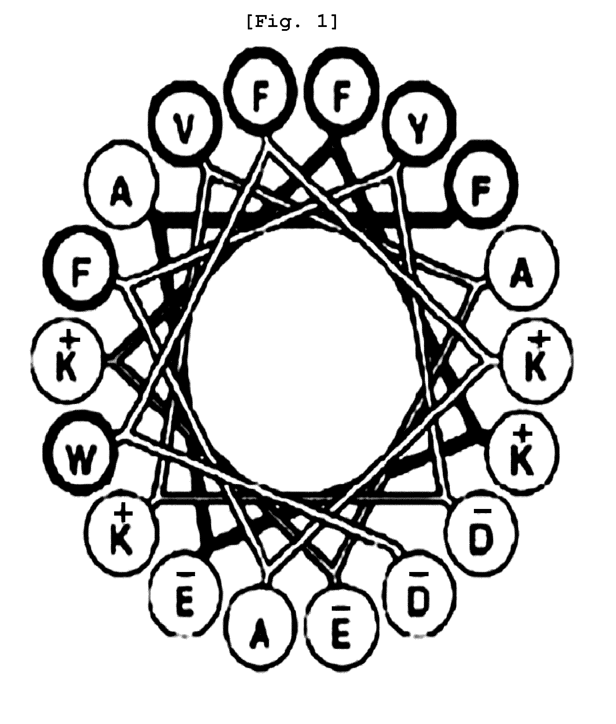

FIG. 1 is a wheel diagram depicting the first generation 4F used in the present invention.

FIG. 2 is a graph showing the cholesterol efflux of peptide series, in which the hydrophilic side residues of the amphiphilic peptide 4F were modified (each peptide was used at a concentration of 20 µM).

FIG. 3 is a graph showing the cholesterol efflux of peptide series, in which the hydrophobic side residues of the amphiphilic peptide 4F were modified (each peptide was used at a concentration of 20 µM).

FIG. 4 is a graph showing the tryptophan fluorescence intensity of the selected peptide under Large Unilamella Vesicles (LUV) conditions. Black color represents the fluorescence intensity of the peptide in a solution, and grey color exhibits the fluorescence intensity of the peptide under a lipid environment (each peptide was used at a concentration of 1 µM, liposome was used at a concentration of 0.1 mg/ml).

FIG. 5 is a graph showing the changes in fluorescence intensity according to the cholesterol composition of the Large Unilamella Vesicle (LUV).

### MODE FOR CARRYING OUT THE INVENTION

The present invention will now be described in further detail by examples.

It would be obvious to those skilled in the art that these examples are intended to be more concretely illustrative and the scope of the present invention as set forth in the appended claims is not limited to or by the examples.

**EXAMPLE 1 SYNTHESIS OF PEPTIDE LIBRARY**

**<1-1> Synthesis of Peptide**

Peptide synthesis was performed by well-informed Fmoc solid phase peptide synthesis method. Fifty mg (0.064 mmol) of Rink Amide resin (Novabiochem) was placed in a vessel, to which 1 ml of methylene chloride was added to inflate thereof. The mixture was inflated by adding 1 ml of DMF (dimethylformamide) for 5 minutes. Deprotection of the resin was performed using 1 ml of 20% piperidine (in DMF) for 5 minutes (three times), followed by washing with 1 ml of DMF five times. Six equivalents of the Fmoc-deprotected amino acid was reacted with a solution containing six equivalents (198 mg) of each PyBop [(benzotriazol-1-yloxy)tripyrrolidinophosphonium hexafluorophosphate] and DIPEA (diisopropylethylamine) for at least 60 minutes. Upon completion of the reaction, the reactant was washed with 1 ml of DMF three times. TNBS test was performed to confirm whether the reaction was successfully done. Particularly, one drop of 10% DIPEA dissolved in DMF and one drop of 2,4,6-trinitrobenzenesulfonic acid (TNBS) dissolved in DMF were dropped on the sample. When it was colorless, it meant reaction was completed. Once amide binding was completed, Fmoc-amino acids having designed sequence were used stepwise 18 times. As a result, 18-mer peptide was synthesized. The peptide was deprotected again before adding 1 ml of 90% DMF and 10% of methylene chloride solution mixture containing 6 equivalents of acetic anhydride and N-hydroxybenzotriazole (HOBt) respectively, and reacting for 45 min or longer to add an acetyl group at the N-terminal of the peptide. After acetylating the N-terminal of the peptide, the resin was washed with 1 ml of DMF and 1 ml of methanol three times each, followed by vacuum-drying. Fifty mg of the resin containing the peptide synthesized by the solid phase peptide synthesis method was loaded in 1 ml of cleavage solution [2.5% TIS (triisopropylsilane), 2.5% water, 95% TFA (trifluoroacetic acid)], followed by stirring for 2 hours. As a result, the synthesized peptide was separated from the resin. The resin was filtered and excessive TFA was eliminated by using nitrogen. Fifty ml of n-hexane:diethyl ether (V:V=1:1) solution cooled at 0 °C in advance was added thereto to elute the synthesized peptide.

**<1-2> Purification of Peptide**

The eluted peptide from Example <1-1> was dissolved in dimethyl sulfoxide, followed by purification by HPLC using C18 column. Water containing 0.1% TFA and acetonitrile were used as HPLC solvents. Particularly, crude peptide was dissolved in dimethyl sulfoxide at the concentration of 10 mg/ml. 100 µl of the solution was loaded in HPLC, followed by purification of the peptide with changing the composition of the solvent from 5% acetonitrile to 70% acetonitrile for 40 minutes. At that time, flow rate was 3 ml/min and detection wavelength was 220 nm. The peptide was recovered at the time point of 20-30 minutes, and vacuumed to evaporate acetonitrile, followed by freeze-drying. The purified acridine-alpha-helical peptide complex was identified by measuring the molecular weight using MOLDI-TOF mass spectrometry.

As a result, the sequence of the synthesized peptide, calculated mass value and actual mass value measured after synthesis and purification are shown in Table 1.

**Table 1**

| SEQ. ID | Reference Name | Amino acid sequences | Mass (M+H⁺) | |
|---|---|---|---|---|
| | | | Calculated | Measured |
| 1 | 4F | Ac-DWFKAFYDKVAEKFKEAF | 2310 | 2312.2 |
| 2 | Sc-4F | Ac-DWFAKDYFKKAFVEEFAK | 2309.1 | 2311.2 |
| 3 | 1a | Ac-EWFKAFYDKVAEKFKEAF | 2323.1 | 2324.2 |
| 4 | 1b | Ac-AadWFKAFYDKVAEKFKEAF | 2337.2 | 2339.9 |
| 5 | 1c | Ac-DWFOrnAFYDKVAEKFKEAF | 2295.1 | 2397 |
| 6 | 1d | Ac-DWFDabAFYDKVAEKFKEAF | 2281.1 | 2282.1 |
| 7 | 1e | Ac-DWFDapAFYDKVAEKFKEAF | 2267.1 | 2268 |
| 8 | 1f | Ac-EWFKAFYEKVAEKFKEAF | 2337.2 | 2338.8 |
| 9 | 1g | Ac-EWFKAFYAadKVAEKFKEAF | 2351.2 | 2353.1 |
| 10 | 1h | Ac-EWFKAFYDOrnVAEKFKEAF | 2309.1 | 2310.9 |
| 11 | 1i | Ac-EWFKAFYDDabVAEKFKEAF | 2295.1 | 2297 |
| 12 | 1j | Ac-EWFKAFYDDapVAEKFKEAF | 2281.1 | 2282.9 |
| 13 | 1k | Ac-EWFKAFYDKVADKFKEAF | 2309.1 | 2309.5 |
| 14 | 1l | Ac-EWFKAFYDKVAAadKFKEAF | 2337.1 | 2337.2 |
| 15 | 1m | Ac-EWFKAFYDKVAEOrnFKEAF | 2309.1 | 2310.6 |
| 16 | 1n | Ac-EWFKAFYDKVAEDabFKEAF | 2295.1 | 2295.4 |
| 17 | 1o | Ac-EWFKAFYDKVAEDapFKEAF | 2281.1 | 2281.3 |
| 18 | 1p | Ac-EWFKAFYDKVAEKFKDAF | 2309.1 | 2310.3 |
| 19 | 1q | Ac-EWFKAFYDKVAEKFKAadAF | 2337.2 | 2337.9 |
| 20 | 1r | Ac-EWFKAFYDKVAEKFOrnEAF | 2309.1 | 2310.4 |
| 21 | 1s | Ac-EWFKAFYDKVAEKFDabEAF | 2295.1 | 2298.8 |
| 22 | 1t | Ac-EWFKAFYDKVAEKFDapEAF | 2281.1 | 2281.9 |
| 23 | 1u | Ac-EWFDabAFYDKVAEKFKEAF | 2295.1 | 2296 |
| 24 | 1v | Ac-EWFDabAFYDKVAEKFDabEAF | 2281.1 | 2281.9 |
| 25 | 2a | Ac-EWAKAFYDKVAEKFKEAF | 2247.1 | 2247.9 |
| 26 | 2b | Ac-EWFKAAYDKVAEKFKEAF | 2247.1 | 2247 |
| 27 | 2c | Ac-EWFKAFYDKVAEKAKEAF | 2247.1 | 2247.9 |
| 28 | 2d | Ac-EWFKAFYDKVAEKFKEAA | 2247.1 | 2246.9 |
| 29 | 2e | Ac-EWWKAFYDKVAEKFKEAF | 2362.1 | 2363.4 |
| 30 | 2f | Ac-EWFKAWYDKVAEKFKEAF | 2362.1 | 2363.3 |
| 31 | 2g | Ac-EWFKAFYDKVAEKWKEAF | 2362.1 | 2363.4 |
| 32 | 2h | Ac-EWFKAFYDKVAEKFKEAW | 2362.1 | 2363.4 |
| 33 | 2i | Ac-EWNal₁KAFYDKVAEKFKEAF | 2373.2 | 2374.4 |
| 34 | 2j | Ac-EWFKANal₁YDKVAEKFKEAF | 2373.2 | 2374.3 |
| 35 | 2k | Ac-EWFKAFYDKVAEKNal₁KEAF | 2373.2 | 2374.6 |
| 36 | 2l | Ac-EWFKAFYDKVAEKFKEANal₁ | 2373.2 | 2374.4 |
| 37 | 2m | Ac-EWNal₂KAFYDKVAEKFKEAF | 2373.2 | 2374.4 |
| 38 | 2n | Ac-EWFKANal₂YDKVAEKFKEAF | 2373.2 | 2374.3 |
| 39 | 2o | Ac-EWFKAFYDKVAEKNal₂KEAF | 2373.2 | 2374.6 |
| 40 | 2p | Ac-EWFKAFYDKVAEKFKEANal₂ | 2373.2 | 2374.4 |
| 41 | 2q | Ac-EWNal₁KAFYDKVAEKFKEANal₂ | 2423.2 | 2423 |
| 42 | 2r | Ac-EWFKANal₁YDKVAEKFKEANal₂ | 2423.2 | 2423 |
| 43 | 2s | Ac-EWNal₂KAFYDKVAEKFKEANal₂ | 2423.2 | 2423 |
| 44 | 2t | Ac-EWFKANal₂YDKVAEKFKEANal₂ | 2423.2 | 2423 |

**EXAMPLE 2 Analysis of the cholesterol efflux level by Apo A-I mimic peptides**

In order to screen which of the peptide synthesized from Example 1 mimics the Apo A-I, cholesterol efflux assay was performed by treating the macrophage cell line with the peptides. In particular, DMEM (Dulbecco's Modified Eagle medium) containing penicillin-streptomycin and 10% FBS (Fetal Bovine Serum) was used as the cell culture medium. Macrophage cell was plated on the 24-well culture plate at the density of 5 x 10⁴ cells numbers per well and then incubated for 24 hr at 37 °C with 5% CO₂. The cell culture medium was replaced with 0.3 ml of fresh DMEM medium containing penicillin-streptomycin and 10% LPDS (Lipoprotein deficient serum) and then incubated for 12-16 hr at 37 °C with 5% CO₂. After the incubation, the cell culture medium was removed and the macrophage cells were washed by adding 1 X PBS (phosphate buffered saline) and shaking. Next, 0.3 ml of subculture medium containing 1 µCi/ml of ³H-labeled cholesterol and 50 µg/ml of Ac-LDL were added into each well and incubated at 37 °C with 5% CO₂. After incubating for 24 hr, the culture medium was removed and the cells were washed with 1 X PBS for 3 times before incubating for 1 hr in 1% BSA (bovine serum albumin). Next, 20 µM of peptides without cytotoxicity were dissolved in fresh DMEM medium containing penicillin-streptomycin. In each well, 0.3 ml of medium was added and further incubated for 4 hr. As a negative control group of 4F, amino acid sequence of 4F synthesized in reverse direction (Sc-4F) was used. The culture medium and the cells were separated to measure the radioactivity using Liquid Scintillation Counter. The level of cholesterol efflux was determined by measuring the ratio of isotope labeled cholesterol remained in the cell and in the cell culture medium, respectively.

According to the result shown in FIG. 2, groups treated with 20 µM of peptide with their hydrophilicity modified (SEQ.ID. NO:3-NO:24) did not show great effect when compared to peptide 4F. Among the peptides with modification in the hydrophilic face, peptide 1a (SEQ. ID. No: 3) had most significant effect, showing 150% of increase in cholesterol efflux when compared to the first generation peptide 4F. The rest of the peptides showed similar effect as peptide 4F (FIG. 2). However, as shown in FIG. 3, the groups treated with 20 µM of peptide with their hydrophobic face modified (SEQ.ID. NO: 25-NO: 44) showed significant difference when compared to hydrophilic face modified peptides. There was a significant reduction in cholesterol efflux in 4 peptides, in which the phenylalanine was substituted with alanine. Peptide 2S, in which the phenylalanine is substituted with bigger sized 2-naphthylalanie showed about 300% increase in cholesterol efflux, when compared to peptide 4F. There was a significant effect when phenylalanine at position #3 was substituted with tryptophan or 2-2-naphthylalanie (FIG. 3)

**EXAMPLE 3 Analyzing the recognition between the Apo-I mimic peptide and the hydrophobic molecule**

**<3-1> Preparation of Large Unilamellar Vesicles (LUVs)**

Total 10 mg of lipid compound was prepared by mixing phosphatidylcholine (soybean) and the LUVs containing cholesterol at 0%, 10%, 20%, 30% and 40%. The lipid compound was dissolved in 2 mL of solution containing 2:1 volumes of chloroform and methanol. The solvent is removed by using distillation condensation device and yielding a thin lipid film in the flask. The lipid film is hydrated by adding 1 mL of tertiary distilled water. Liposomes of the same size were generated by extruding the solution through a 0.2 polycarbonate filter for 5 times, and 0.1 µm polycarbonate filter for 5 times using a cylinder fitted with a high pressure regulator. The amount of cholesterol contained in the liposome was measured by using cholesterol Lab-assay kit.

**<3-2> Tryptophan fluorescence measurement**

Fluorescence Anisotropy was measured using AMINCO-Bowman Series Luminescence Spectrometer at 20 °C. For the buffer system, 10 mM HEPES (N-2-Hydroxyethylpiperazine-N'-2-Ethanesulfonic Acid, pH 7.4) containing 140 mM NaCl, 1 mM EDTA (ethylenediaminetetraacetic acid) was used. The concentration of the peptide was 500 µM and was kept at 0 °C. The fluorescence emission spectrum was monitored by adding 1 µl of peptide with 499 µl buffer solution to a final concentration of 1 µM. Liposome was added to the above buffer and peptide mixture solution to the final concentration of 0.1 mg/ml. The ratio of the intensity was calculated by measuring the spectrum in the condition of with or without liposome.

According to the result shown in FIG.4, the tryptophan fluorescence intensity measured under LUV condition was about 1.9 and 2.1 for 4F and 1a, respectively, however, the fluorescence intensity reached about 4.3 for 2s. There was a dramatic increase in fluorescence intensity in hydrophobic face modified peptide 2s, when compared to 4F or hydrophilic face modified peptide 1a. A rapid shift of the wavelength to shorter wavelength was also observed. (FIG.4).

In addition, as shown in FIG.5, the tryptophan fluorescence intensity was measured by using the liposome prepared by increasing the relative amount of cholesterol. Peptides 4F and sc-4F showed fluorescence intensity that was irrelevant to the concentration of cholesterol, however, 2s peptide showed significant increase of fluorescence intensity according to the increased concentration of the cholesterol inside the liposome. Therefore, the result suggests that peptide 2s had a high recognition for cholesterol (FIG.5).

### INDUSTRIAL APPLICABILITY

As described above, the Apo A-I mimic peptide of the present invention has superior treatment effect for hyperlipidemia than existing peptides, therefore it can be effectively used as a candidate peptide for developing and manufacturing a therapeutic agent for hyperlipidemia and related diseases.

The development of a new drug for treating hyperlipidemia by increasing HDL is at risk since the failure of CETP inhibitors. CETP inhibitor may have functions other than converting HDL to LDL, therefore it is difficult to use CETP and CETP inhibitors as the target and target inhibitor for increasing HDL unless a solution is found.

The only currently available target is the Apo A-I mimic protein or peptide. D-4F, which is a precursor material has raised several concerns and stalled phase 1 clinical trials. Therefore, it is an ideal time for developing the second and third generation Apo A-I mimic peptides, which has improved efficacy when compared to D-4F. The novel peptide disclosed in the present invention has 200-300% higher treatment effect in biochemical and biological aspects, and is therefore a suitable therapeutic agent for hyperlipidemia.

## Claims

1. An amphipathic peptide library comprising amphipathic peptide sequence containing 3 to 8 phenylanines (F) at one side of the amphipathic α-helical peptide, wherein a positively charged amino acid of the hydrophilic amino acid is substituted with an amino acid with fewer carbon numbers, and a negatively charged amino acid is substituted with an amino acid with more carbon numbers.

2. The amphipathic peptide library as set forth in claim 1, wherein the positively charged substituted amino acid contains an amine functional group, and the negatively charged substituted amino acid contains an acid functional group.

3. The amphipathic peptide library as set forth in claim 1, which comprises amphipathic peptide containing amino acid sequence (4F) represented by SEQ ID NO: 1, wherein one or two of the hydrophilic amino acid lysine (K) is substituted with ornithine (Orn), 1,4-diaminobutyric acid (Dab) and 1,3-dipropanoic acid (Dap), and one or two of the amino acid glutamic acid (E) or aspartic acid (D) is substituted with glutamic acid or amino adipic acid (Aad)

4. The amphipathic peptide library as set forth in claim 3, wherein the amphipathic peptide comprises one or more peptide containing amino acid sequences represented by SEQ. ID. NOs: 3 to 24.

5. An amphipathic peptide library comprising amphipathic peptide sequence containing 3 to 8 phenylanines (F) at one side of the amphipathic α-helical peptide, wherein one or more phenylalanine of the hydrophobic amino acid is substituted with an aromatic amino acid other than phenylalanine.

6. The amphipathic peptide library as set forth in claim 5, wherein the aromatic amino acid other than phenylalanine is any one selected from the group consisting of phenylalanine derivatives, tryptophan and its derivatives, and napthylalanine and its derivatives.

7. The amphipathic peptide library as set forth in claim 5, which comprises amphipathic peptide containing one or two of the hydrophobic amino acid phenylalanine (F) of the amino acid sequence represented by SEQ. ID. NO: 1 is substituted with alanine (A), tryptophan (W), 1-naphtyhlalanine (Nal1) or 2-naphtyhlalanine (Nal2).

8. The amphipathic peptide library as set forth in claim 7, wherein the amphipathic peptide comprises one or more peptide containing amino acid sequences represented by SEQ. ID. NOs: 25 to NO: 44.

9. A method of screening Apo A-I mimic peptide, the method comprising the steps of:
1) preparing the amphipathic peptide library of claim 1 or claim 5;
2) analyzing cholesterol efflux in a cell by the amphipathic peptide library; and
3) selecting peptide which shows increased level of cholesterol efflux when compared to Apo A-I.

10. The method as set forth in claim 9, wherein the cell of step 1) is a macrophage cell.

11. The method as set forth in claim 9, wherein analyzing cholesterol efflux of step 2) is by using cholesterol efflux assay.

12. A method of screening Apo A-I mimic peptide, which comprises the steps of:
1) preparing the amphipathic peptide library of claim 1 or claim 5; and
2) selecting peptide which shows increased level of tryptophan fluorescence intensity when compared to Apolipoprotein A-1(Apo A-I) by mixing a tryptophan fluorescence probe molecule to the amphipathic peptide library of step 1) and detecting with a fluorescence spectrometer.

13. A high density lipoprotein (HDL) enhancing agent comprising the amphipathic peptide selected according to the method of claim 9 or claim 12, as an active component.

14. A therapeutic agent or diagnostic reagent for hyperlipidemia and related diseases comprising the amphipathic peptide selected according to the method of claim 9 or claim 12 as an active component.

15. The therapeutic agent or diagnostic reagent as set forth in claim 14, wherein the hyperlipidemia and related diseases is any one selected from the group consisting of hypercholesterolemia, atherosclerosis, coronary artery disease, cardiovascular disease, restenosis, high blood pressure, angina, diabetes, obesity, Alzheimer's disease and multiple sclerosis.

16. A method of treating hyperlipidemia and related diseases comprising a step of administering the therapeutic agent of claim 14 to a subject.

17. A method of diagnosing hyperlipidemia and related diseases comprising a step of administering the therapeutic agent of claim 14 to a subject.

18. A use of an amphipathic peptide for manufacturing therapeutic agent or diagnostic reagent for hyperlipidemia and related diseases, which comprises amphipathic peptide sequence containing 4 phenylanines (F) at the amphipathic α-helical peptide, wherein a positively charged amino acid of the hydrophilic amino acid is substituted with an amino acid with fewer carbon numbers, and a negatively charged amino acid is substituted with an amino acid with more carbon numbers,

19. A use of an amphipathic peptide for manufacturing therapeutic agent or diagnostic reagent for treating hyperlipidemia and related diseases, which comprises amphipathic peptide sequence containing 4 phenylanines (F) at the amphipathic α-helical peptide, wherein one or more phenylalanine of the hydrophobic amino acid is substituted with aromatic amino acid other than phenylalanine,
